# FASCICULE DE BREVET EUROPEEN

(11) **EP 0 699 657 B1**
(45) Date de publication et mention de la délivrance du brevet: **27.03.2002**
(21) Numéro de dépôt: 95401831.3
(22) Date de dépôt: 04.08.1995
(51) Int. Cl.: C07C 263/10

(54) **Procédé de préparation de composés du type polyisocyanates aromatiques en phase gazeuse**
Verfahren zur Herstellung von aromatischen Polyisocyanatverbindungen in der Gasphase
Process for the preparation of aromatic polyisocyanate compounds in gas phase

(30) Priorité: 12.08.1994 FR 9410009
(43) Date de publication de la demande: 06.03.1996
(73) Titulaire: RHODIA CHIMIE, 92512 Boulogne Billancourt Cedex (FR)
(72) Inventeur: Armand, Jérôme, F-69006 Lyon (FR); Revelant, Denis, F-69740 Genas (FR); Chiarelli, Henri, F-69360 Communay (FR); Vacus, Pascal, F-69390 Millery (FR)
(74) Mandataire: Dubruc, Philippe

(56) Documents cités:
- EP-A- 0 570 799
- EP-A- 0 593 334
- DATABASE WPI Week 7438 Derwent Publications Ltd., London, GB; AN 74-67320V & SU-A-407 567 (VUKHTIN N. N. ET AL.)

## Description

La présente invention concerne un procédé de préparation de composés aromatiques substitués par au moins deux groupements isocyanates. Elle concerne plus particulièrement un procédé de préparation de toluène diisocyanate, ses isomères seuls ou en mélange.

La préparation de composés aromatiques substitués par un ou plusieurs groupements isocyanates par réaction d'amines avec du phosgène en phase gazeuse est connue depuis longtemps, bien qu'elle n'ait eu de véritable essor que pour la transformation d'amines monofonctionnelles.

L'inconvénient majeur des procédés connus ayant pour objet la formation de polyisocyanates aromatiques, est qu'ils mettent oeuvre des quantités très importantes de phosgène par rapport aux fonctions amines engagées dans la réaction. En effet, la formation de polyisocyanates à partir des polyamines correspondantes, nécessite un excès de phosgène de l'ordre de 200 à 300 % en moles par rapport aux fonctions amine. En effet il est connu que de tels excès de phosgène permettent d'augmenter la vitesse de la phosgénation. Par ailleurs, ils évitent la formation de sous-produits indésirables, résultant de la réaction de l'amine avec l'isocyanate, et qui, hormis le fait de diminuer le rendement en polyisocyanate formé, présentent aussi bien souvent l'inconvénient d'être solides et de causer des bouchage des réacteurs. De tels problèmes n'existent pas lorsque les amines mises en jeu sont des monoamines aliphatiques ou aromatiques, ou des polyamines aliphatiques.

Cependant, si des excès importants de phosgène sont préconisés pour l'efficacité de la réaction, il n'est pas souhaité de travailler avec de telles quantités, sur le plan de la sécurité, du fait de la toxicité de ce réactif et des contraintes imposées avec l'utilisation de ce dernier ; contraintes d'autant plus importantes que l'encours en phosgène dans l'installation est élevé.

Une autre particularité des procédés de réaction de polyamines en phase gazeuse avec le phosgène réside dans la nécessité, pour obtenir de bons rendements en polyisocyanates, d'augmenter l'efficacité du mélange entre les réactifs afin d'éviter les réactions parasites.

Ainsi, dans ce but, on a préconisé l'emploi de réacteurs comprenant des moyens mobiles d'agitation du mélange réactionnel. Cependant cette méthode présente des inconvénients liés à l'utilisation de pièces tournantes de l'agitateur mécanique, tels que notamment des problèmes d'étanchéité au niveau de l'axe de rotation, des problèmes d'encrassement et donc de blocage du mobile d'agitation dus à des sous-produits de réactions collants.

La demande de brevet EP 570 799 ne décrit pas l'emploi d'un réacteur du type précité mais requiert d'injecter les réactifs dans une zone de mélange statique de telle sorte que le temps de séjour y soit de 0,1 à 0,3 seconde et que le degré de ségrégation entre les réactifs soit de 10⁻³. Une fois l'homogénéisation effectuée, le mélange réactionnel entre dans une zone de réaction proprement dite, sans rétro-mélange, dans laquelle l'écoulement est du type piston et caractérisée par un nombre de Bodenstein et/ou un nombre de Reynolds minimal. Ces caractéristiques sont essentielles car la description indique qu'à défaut d'être vérifiées, on constate des phénomènes de bouchage du réacteur. L'inconvénient de ce type de procédé est qu'il nécessite la mise en oeuvre d'excès de phosgène importants car variant de 150 à 250 % en moles, rapporté aux fonctions amine.

La présente invention a donc pour objet un procédé concernant la préparation de composés aromatiques substitués par au moins deux groupements isocyanates, ne présentant pas les inconvénients précédemment indiqués, notamment en ce qui concerne l'excès de phosgène.

Ainsi, selon une première variante de l'invention, le procédé est caractérisé en ce que l'on met en contact, dans un réacteur mixte comprenant une première zone, de forme cylindrique, du type parfaitement agité, et dont la section est voisine de la longueur, correspondant à 20 à 80 % du volume total du réacteur, et une seconde zone, de forme tubulaire, dans laquelle l'écoulement du flux y est voisin du type piston, et dont la longueur est supérieure à la section, correspondant à 80 à 20 % du volume total du réacteur, au moins un composé (A) comprenant au moins deux fonctions amine primaire et au moins un motif ammmatique, avec du phosgéne, les deux réactifs étant introduits en phase gazeuse, en présence d'un excès de phosgène, rapporté au nombre de moles de fonctions amine du composé (A), compris entre 0 et 100 %.

Selon une seconde caractéristique de l'invention, le procédé est caractérisé par le fait l'on met en contact dans un réacteur mixte comprenant une première zone du type parfaitement agité correspondant à 20 à 80 % du volume total du réacteur, et une seconde zone dans laquelle l'écoulement du flux y est voisin du type piston, correspondant à 80 à 20 % du volume total du réacteur ; le temps de séjour dans l'une et l'autre des zones est proportionnel à la répartition volumique de chacune des deux zones, au moins un composé (A) comprenant au moins deux fonctions amine primaire et au moins un motif aromatique, avec du phosgène, les deux réactifs étant Introduits en phase gazeuse, en présence d'un excès de phosgène, rapporté au nombre de moles de fonctions amine du composé (A), compris entre 0 et 100 %.

Le procédé de préparation selon l'invention permet donc d'obtenir les isocyanates correspondant à la transformation des amines précitées, sans qu'il soit nécessaire d'effectuer la réaction en présence d'un très fort excès en phosgène. On a en effet constaté que l'on pouvait mettre en oeuvre la réaction de phosgénation de polyamines aromatiques dans ces conditions, sans par ailleurs nécessiter une augmentation rédhibitoire du temps de réaction. En outre, le rendement en isocyanate en sortie de réacteur est aussi élevé que celui obtenu avec les procédés mettant en oeuvre un excès de phosgène important

Un avantage important du procédé selon l'invention est qu'il n'est plus nécessaire de recycler le phosgène n'ayant pas réagi, contrairement aux procédés mettant en oeuvre de forts excès en ce composé, qui requièrent une telle opération pour être rentables économiquement. Eviter le recyclage du phosgène en excès présente rentables économiquement. Eviter le recyclage du phosgène en excès présente l'avantage de pouvoir s'affranchir d'une zone du procédé dans laquelle circule une quantité importante de phosgène concentré, voire sous pression, avec toutes les contraintes de sécurité que cela suppose.

Mais d'autres avantages et caractéristiques apparaîtront plus clairement à la lecture de la description des exemples qui vont suivre.

Ainsi qu'il a été dit plus haut, le procédé selon l'invention comprend la mise en contact, en phase gazeuse, d'au moins un composé (A) présentant au moins deux fonctions amine primaire et au moins un motif aromatique, avec du phosgène.

Selon un mode particulier de réalisation de l'invention, on met en oeuvre le procédé en utilisant au moins un composé (A) comprenant au moins deux fonctions amine primaire et au moins un motif aromatique en C₆-C₁₄, de préférence en C₆-C₁₀, substitué ou non par un ou plusieurs radicaux hydrocarbonés, saturés ou non, linéaires, cycliques ou ramifiés en C₁-C₁₀.

Plus précisément, les radicaux hydrocarbonés précités, substituant éventuellement lesdits motifs aromatiques, peuvent être choisis parmi les radicaux alkyles, aryles, alkyle-aryles et aryle-alkyles en C₁-C₁₀, de préférence en C₁-C₆.

De préférence, le composé (A) correspond au produit de formule:

H₂N-R-NH₂ (1),

formule (1) dans laquelle R représente le motif aromatique, substitué ou non, décrit ci-dessus.

Selon un mode plus particulier de réalisation de l'invention, on utilise au moins un composé (A) dans lequel le motif R précité est éventuellement substitué par un ou plusieurs radicaux alkyles en C₁-C₁₀, de préférence en C₁-C₆.

Comme motif R, on peut notamment citer les noyaux benzénique et naphtalénique substitués ou non par un ou plusieurs radicaux méthyle, éthyle, propyle, butyle, pentyle, hexyle et/ou leurs isomères.

De façon préférée, le procédé est mis en oeuvre en utilisant au moins un composé (A) choisi parmi la toluène diamine, la xylylène diamine, la phénylène diamine ; ces composés pouvant être utilisés seuls ou en mélange, avec ou sans leurs isomères

Le composé (A), de façon encore plus préférée, est la toluène diamine.

Selon une caractéristique de l'invention, la réaction est effectuée en présence d'un excès de phosgène, rapporté au nombre de fonctions amines dans le composé (A) variant entre 0 et 100 % en mole.

Un mode particulièrement avantageux consiste à mettre en oeuvre la réaction en présence d'un excès de phosgène variant entre 5 et 60 % en mole, rapporté à la même base que précédemment.

Les réactifs mis en contact dans la réaction selon l'invention, c'est-à-dire au moins un composé (A) et le phosgène, peuvent être engagés seuls ou en présence d'un diluant. Par diluant, on entend un composé inerte vis-à-vis des réactifs et des produits de la réaction, dans les conditions réactionnelles.

Outre les gaz inertes, comme notamment l'azote, on peut également utiliser, en tant que diluant, la vapeur d'un solvant du composé (A) et/ou du phosgène. Le solvant précité peut notamment être choisi parmi le benzène, le xylène, l'orthodichlorobenzène, le monochlorobenzène, ou tout autre solvant employé habituellement dans les réactions de phosgénation, dans la mesure où ils résistent dans les conditions de la réaction (température, temps de séjour).

Dans le cas où le composé (A) est solubilisé dans un diluant, celui-ci se trouve présent à une concentration massique comprise plus particulièrement entre 3 et 30 % dans le diluant. De préférence, cette concentration est comprise entre 10 et 20 %.

Ainsi, si un diluant est utilisé avec l'amine, le mélange a lieu de préférence avant l'introduction des réactifs dans le réacteur. On effectue en général au préalable la solubilisation, en phase liquide, de l'amine dans son solvant. Le mélange résultant est ensuite vaporisé à la température requise pour la réaction, par tout moyen connu de l'homme du métier.

Les réactifs, en présence le cas échéant d'un diluant, sont mis en contact en phase vapeur. Les réactifs sont donc préchauffés par tout moyen connu, de façon à obtenir des produits vaporisés lors de leur introduction dans le réacteur. Par ailleurs, la réaction est menée dans des conditions telles que produits et réactifs restent sous forme de vapeur.

Habituellement, la température de préchauffage des réactifs est du même ordre de grandeur que celle requise pour effectuer la phosgénation.

Selon une autre caractéristique de la présente invention, la réaction est mise en oeuvre dans un réacteur mixte comprenant une première zone du type parfaitement agité, correspondant à 20 à 80 % du volume total du réacteur, et une seconde zone dans laquelle l'écoulement du flux y est voisin du type piston, correspondant à 80 à 20 % du volume total du réacteur.

La première zone comprend plus particulièrement un réacteur dont la longueur est du même ordre de grandeur que sa section. Pour des raisons évidentes de commodité, le réacteur de cette première zone présente une forme cylindrique. Mais bien entendu, tout autre forme géométrique (cubique, sphérique) dont la section est voisine de la longueur convient à l'invention.

Selon un mode de réalisation préféré de l'invention, la première zone correspond à 40 à 80 % du volume total du réacteur.

Les réactifs sont introduits dans des conditions telles que la zone du réacteur corresponde à un réacteur proche du type parfaitement agité.

Dans ce but, l'introduction des réactifs doit de préférence avoir lieu de façon à promouvoir un régime turbulent dans cette zone et de façon à avoir une bonne homogénéisation du mélange réactionnel.

L'introduction peut donc avoir lieu de différentes manières. De préférence, on emploie des moyens favorisant le rétromélange des réactifs (ou mélange en retour). On peut citer par exemple, et sans intention de se limiter, les buses ou les injecteurs multijets.

Les buses peuvent notamment être du type concentriques, c'est-à-dire être composées de deux tubes concentriques insérés l'un dans l'autre formant une partie centrale et une partie annulaire. Le composé (A) et le phosgène, éventuellement en présence d'un diluant, peuvent indifféremment introduits par la partie annulaire ou par la partie centrale.

Les injecteurs multijets peuvent comporter soit un système comprenant plusieurs jets convergeant vers un ou plusieurs jets centraux, soit plusieurs jets situés dans différents endroits du réacteur avec des angles et des directions de pulvérisation différentes, permettant le brassage du flux gazeux.

L'introduction des réactifs, sous forme vaporisée, peut avoir lieu dans toute partie de la zone agitée. Ainsi, on peut introduire les réactifs dans une région proche de la paroi jusqu'à une région proche du centre du réacteur.

Cette première zone peut ou non comprendre des systèmes statiques (obstacles, chicanes) favorisant le rétromélange des réactifs et l'homogénéisation du mélange.

La seconde zone du réacteur mis en oeuvre dans le procédé selon l'invention est caractérisée par le fait que l'écoulement y est voisin du type piston. Cette zone correspond à 80 à 20 % du volume total du réacteur.

Cette zone du réacteur correspond plus particulièrement à un finisseur pour la réaction de phosgénation. Ainsi, elle permet d'atteindre un taux de conversion maximal en minimisant le volume total du réacteur.

Elle comprend un réacteur dont la longueur est supérieure à la section. Les réacteurs sous forme de tube en sont un exemple, bien que d'autres formes géométriques conviennent, dans la mesure où la caractéristique précédente est vérifiée.

Selon un mode de réalisation préféré de l'invention, la seconde zone correspond à 60 à 20 % du volume total du réacteur.

Cette zone du réacteur peut elle aussi être garnie d'obstacles internes ou de chicanes, ou bien de préférence, en être dépourvue.

De préférence le réacteur mis en oeuvre dans la réaction selon l'invention est dépourvu de moyen d'agitation mécaniques mobiles, que ce soit dans la première ou dans la seconde zone.

La réaction selon l'invention peut être réalisée dans tout type de réacteur dont le matériau est compatible avec les conditions opératoires. Ainsi, la réaction peut être réalisée notamment dans un réacteur dont le matériau est du verre ou de l'acier, allié ou émaillé.

Le temps de séjour des réactifs dans le réacteur comprenant les deux zones est, plus particulièrement compris entre 1,5 et 30 secondes. De préférence, le temps de séjour est compris entre 3 et 15 secondes. Il est à noter que le temps de séjour dans l'une et l'autre des deux zones est proportionnel à la répartition volumique de chacune des deux zones par rapport à l'ensemble.

Il a été trouvé d'une façon surprenante que le fait de mettre en oeuvre une partie non négligeable de la réaction dans un réacteur de type parfaitement agité n'apportait pas les inconvénients normalement attendus, ceci malgré un faible excès de phosgène.

Ceci est d'autant plus surprenant que les procédés connus préconisent de mélanger les réactifs pendant une durée aussi courte que possible, afin d'éviter toute réaction, celle-ci ayant lieu dans une zone différente du réacteur, dans laquelle le temps de séjour est jusqu'à 10 fois plus grand que dans la précédente. La zone dans laquelle la réaction proprement dite a lieu est caractérisée par l'absence de rétromélange des réactifs qui était sensé être à l'origine des bouchages. Or la présente invention a montré qu'il n'en était rien, malgré le fait, que la majeure partie de la réaction de phosgénation a lieu dans un réacteur où existe un tel rétromélange. Par ailleurs, on a constaté que la réaction selon l'invention mise en oeuvre dans un réacteur comprenant deux zones pouvait avoir lieu avec temps de séjour du même ordre de grandeur que les procédés mis en oeuvre dans des réacteurs de type piston.

Enfin, les réacteurs comprenant une partie dans laquelle le mélange est parfaitement agité permet d'améliorer les échanges thermiques et d'éviter la présence de points chauds dans le réacteurs.

La température à laquelle la réaction de phosgénation selon l'invention est effectuée, varie habituellement entre 250 et 500°C. Plus particulièrement ladite température de réaction est comprise entre 300 et 400°C. Par température de réaction, on entend la température régnant dans le réacteur.

Le procédé selon l'invention peut être réalisé indifféremment sous pression, à pression réduite ou à pression atmosphérique. A titre d'exemple, la pression régnant dans le réacteur peut varier entre 0,5 et 1,5 bar absolus. De préférence, on procède à une pression voisine de la pression atmosphérique.

Une fois la réaction de phosgénation réalisée, les produits obtenus ainsi que les réactifs n'ayant pas réagi sont séparés selon toute méthode connue de l'homme du métier.

On pourra par exemple séparer l'isocyanate produit par condensation sélective de celui-ci dans un solvant adéquat.

Pour des questions de commodité, ledit solvant est de préférence choisi de telle sorte que sa température d'ébullition soit supérieure à celle de la décomposition du chlorure de carbamyle correspondant à l'isocyanate formé. Choisir un solvant répondant à ce critère évite une étape ultérieure de décomposition dudit chlorure de carbamyle,

Par ailleurs, ce même solvant doit pouvoir, de préférence, se condenser à une température pour laquelle les produits, comme notamment le phosgène restant et l'acide chlorhydrique formé, restent à l'état gazeux.

L'isocyanate récupéré est alors purifié, notamment par distillation.

En ce qui concerne le phosgène qui n'a pas réagi, il est à noter que les procédés classiques prévoient le recyclage de ce composé à la réaction. En effet, étant donné les excès très importants de phosgène employés, de tels procédés ne sont rentables économiquement que si ce composé est recyclé. Cependant, dans cette zone du procédé se trouve située une grande quantité de phosgène concentré, voire sous pression, ce qui représente un inconvénient très important sur le plan des conditions de sécurité à satisfaire.

Selon un mode de réalisation particulièrement avantageux de l'invention, il n'est plus nécessaire de recycler le phosgène. Ainsi se trouve éliminée une source très importante d'inconvénients due à la présence, dans une zone particulière du procédé, de phosgène concentré.

Selon ce mode particulier, le phosgène séparé des produits de la réaction est détruit par tout moyen connu de l'homme du métier, comme par mise en contact avec une base telle que la soude, ou avec de l'eau.

Quant à l'acide chlorhydrique, celui-ci peut être séparé des produits de la réaction, en vue d'être valorisé (séparation du phosgène présent par absorption, distillation) ou bien détruit avec l'emploi d'une base.

Il est à noter cependant que recycler le phosgène à la réaction est tout à fait possible et ne sortirait pas du cadre de la présente invention.

Dans ce cas particulier, le phosgène peut être séparé de l'acide chlorhydrique par distillation, par absorption dans un solvant à basse température, ou tout autre moyen, puis recyclé dans le procédé de phosgénation.

Des exemples concrets mais non limitatifs de réalisation de l'invention vont maintenant être présentés.

### EXEMPLE 1

On utilise un réacteur cylindrique dont le diamètre est égal à la hauteur et d'un volume de 0,18 I, alimenté par un système d'injecteurs situés à une distance d'un quart de diamètre du centre du réacteur (jets séparés pour la mélange amine/solvant et pour le phosgène) suivi d'un réacteur tubulaire de 0,12 I (longueur de 30 cm).

Le système d'injection est du type de celui décrit dans l'article de MATRAS et VILLERMAUX, Chem. Ing. Science, 1973, 28, 129.

On introduit dans le réacteur :
- 600 g/h d'un mélange gazeux de toluène diamine à 10 % en poids dans l'orthodichlorobenzène vaporisé à 300 °c,
- 155 g/h de phosgène pur préchauffé à 300°C.

La température du mélange réactionnel dans le réacteur est de 320°C et le temps de séjour moyen des réactifs est de l'ordre de 3,2 à 3,9 secondes.

De cette façon et après analyse, on obtient un rendement en toluène diisocyanate supérieur à 95 % en sortie du réacteur et on n'observe pas d'encrassement significatif dans le réacteur après plus d'une heure de fonctionnement.

### EXEMPLE 2

On utilise un réacteur cylindrique dont le diamètre est égal à la hauteur et d'un volume de 0,18 l, alimenté par un système d'injecteurs situés à une distance d'un demi diamètre du centre du réacteur (jets séparés pour la mélange amine/solvant el pour le phosgène) suivi d'un réacteur tubulaire de 0,7 (longueur de 1,4 m).

Le système d'injection est celui décrit dans l'exemple 1.

On introduit dans le réacteur :
- 1100 g/h d'un mélange gazeux de toluène diamine à 10 % en poids dans l'orthodichlorobenzène vaporisé à 330 °C,
- 196 g/h de phosgène pur préchauffé à 330°C.

La température du mélange réactionnel dans le réacteur est de 350°C et le temps de séjour moyen des réactifs est de l'ordre de 5 à 6,5 secondes.

De cette façon et après analyse, on obtient un rendement en toluène diisocyanate supérieur à 95 % en sortie du réacteur et on n'observe pas d'encrassement significatif dans le réacteur après plus d'une heure de fonctionnement.

## Revendications

1. Procédé de préparation de composés aromatiques substitués par au moins deux groupements isocyanates, **caractérisé en ce que** l'on met en contact dans un réacteur mixte comprenant une première zone, de forme cylindrique, du type parfaitement agité, et dont la section est voisine de la longueur, correspondant à 20 à 80 % du volume total du réacteur, et une seconde zone de forme tubulaire, dans laquelle l'écoulement du flux y est voisin du type piston, et dont la longueur est supérieure à la section, correspondant à 80 à 20 % du volume total du réacteur, au moins un composé (A) comprenant au moins deux fonctions amine primaire et au moins un motif aromatique, avec du phosgène, les deux réactifs étant introduits en phase gazeuse, en présence d'un excès de phosgène, rapporté au nombre de moles de fonctions amine du composé (A), compris entre 0 et 100 %.

2. Procédé de préparation de composés aromatiques substitués par au moins deux groupements isocyanates, **caractérisé en ce que** l'on met en contact dans un réacteur mixte comprenant une première zone du type parfaitement agité correspondant à 20 à 80 % du volume total du réacteur, et une seconde zone dans laquelle l'écoulement du flux y est voisin du type piston, correspondant à 80 à 20 % du volume total du réacteur ; le temps de séjour dans l'une et l'autre des zonas est proportionnel à la répartition volumique de chacune des deux zones, au moins un composé (A) comprenant au moins deux fonctions amine primaire et au moins un motif aromatique, avec du phosgène, les deux réactifs étant introduits en phase gazeuse, en présence d'un excès de phosgène, rapporté au nombre de moles de fonctions amine du composé (A), compris entre 0 et 100%.

3. Procédé selon l'une des revendications 1 ou 2, **caractérisé en ce que** l'on effectue la réaction en présence d'un excès de phosgène, rapporté au nombre de fonctions amine du composé (A), compris entre 5 et 60 %.

4. Procédé selon l'une quelconque des revendications précédentes, **caractérisé en ce que** l'on effectue la réaction dans un réacteur mixte dont la première zone correspond à 40 à 80 % du volume total du réacteur et la seconde zone à 60 à 20 % du volume total du réacteur.

5. Procédé selon l'une quelconque des revendications précédentes, **caractérisé en ce que** l'on utilise au moins un composé (A) comprenant au moins deux fonctions amine primaire et au moins un motif aromatique en C₆-C₁₄, de préférence en C₆-C₁₀, substitué ou non par un ou plusieurs radicaux hydrocarbonés, saturés ou non, linéaires, cycliques ou ramifiés en C₁-C₁₀, de préférence en C₁-C₆.

6. Procédé selon la revendication précédente, **caractérisé en ce que** l'on utilise au moins un composé (A) dans lequel le motif aromatique précité peut être éventuellement substitué par un ou plusieurs radicaux hydrocarbonés choisis parmi les radicaux alkyles, aryles, alkyle-aryles et aryle-alkyles en C₁-C₁₀, de préférence en C₁-C₆.

7. Procédé selon l'une quelconque des revendications précédentes, **caractérisé en ce que** l'on utilise au moins un composé (A) correspond au produit de formule :
H₂N-R-NH₂ (1),
formule (1) dans laquelle R représente un motif aromatique tel que décrit dans l'une des revendications 4 ou 5.

8. Procédé selon la revendication précédente, **caractérisé en ce que** l'on utilise au moins un composé (A) de formule (1) dans laquelle R représente un motif aromatique en C₆-C₁₄, de préférence en C₆-C₁₀, substitué ou non par un ou plusieurs radicaux alkyles en C₁-C₁₀, et de préférence en C₁-C₆.

9. Procédé selon la revendication précédente, **caractérisé en ce que** l'on utilise au moins un composé (A) choisi parmi la toluène diamine, la xylylène diamine et la phénylène diamine seuls ou en mélange, avec ou sans leurs isomères.

10. Procédé selon l'une quelconque des revendications précédentes, **caractérisé en ce que** l'on utilise au moins un composé (A) et/ou le phosgène, seuls ou en présence d'un diluant

11. Procédé selon la revendication précédente, **caractérisé en ce que** l'on utilise le ou les composés (A) avec une concentration massique comprise entre 3 et 30%, de préférence entre 10 et 20%, dans le diluant.

12. Procédé selon l'une quelconque des revendications précédentes, **caractérisé en ce que** le temps de séjour des réactifs dans le réacteur est compris antre 1,5 et 30 secondes.

## Patentansprüche

1. Verfahren zur Herstellung von aromatischen Verbindungen, die mit mindestens zwei Isocyanatgruppen substituiert sind, **dadurch gekennzeichnet, daß** man in einem Mischreaktor mindestens eine Verbindung (A), die mindestens zwei primäre Aminfunktionen und mindestens eine aromatische Einheit umfaßt, mit Phosgen in Kontakt bringt, wobei die beiden Reagenzien in der Gasphase in Gegenwart eines Überschusses an Phosgen von 0 bis 100 %, bezogen auf die Anzahl der Mol an Aminfunktionen der Verbindung (A), eingebracht werden, wobei der Mischreaktor eine erste Zone in zylindrischer Form, die vollkommen durchmischt wird und deren Querschnitt etwa ihrer Länge entspricht, wobei diese erste Zone 20 bis 80 % des Gesamtvolumens des Reaktors entspricht, und eine zweite Zone mit rohrförmiger Form umfaßt, in der die Strömung des Flusses etwa kolbenartig ist und deren Länge größer als ihr Querschnitt ist, wobei diese zweite Zone 80 bis 20 % des Gesamtvolumens des Reaktors entspricht.

2. Verfahren zur Herstellung von aromatischen Verbindungen, die mit mindestens zwei Isocyanatgruppen substituiert sind, **dadurch gekennzeichnet, daß** man in einem Mischreaktor mindestens eine Verbindung (A), die mindestens zwei primäre Aminfunktionen und mindestens eine aromatische Einheit umfaßt, mit Phosgen in Kontakt bringt, wobei die beiden Reagenzien in der Gasphase in Gegenwart eines Überschusses an Phosgen von 0 bis 100 %, bezogen auf die Anzahl der Mol an Aminfunktionen der Verbindung (A), eingebracht werden, wobei der Mischreaktor eine erste Zone, die vollkommen durchmischt wird und die 20 bis 80 % des Gesamtvolumens des Reaktors entspricht, und eine zweite Zone, in der die Strömung des Flusses etwa kolbenartig ist und die 80 bis 20 % des Gesamtvolumens des Reaktors entspricht, wobei die Verweildauer in beiden Zonen jeweils proportional zu Volumenverteilung dieser beiden Zonen ist.

3. Verfahren nach Anspruch 1 oder 2, **dadurch gekennzeichnet, daß** man die Reaktion in Gegenwart eines Überschusses an Phosgen von 5 bis 60 %, bezogen auf die Anzahl der Aminfunktionen der Verbindung (A), durchführt.

4. Verfahren nach einem der vorhergehenden Ansprüche, **dadurch gekennzeichnet, daß** man die Reaktion in einem Mischreaktor durchführt, dessen erste Zone 40 bis 80 % des Gesamtvolumens des Reaktors entspricht und dessen zweite Zone 60 bis 20 % des Gesamtvolumens des Reaktors entspricht.

5. Verfahren nach einem der vorhergehenden Ansprüche, **dadurch gekennzeichnet, daß** man mindestens eine Verbindung (A) einsetzt, die mindestens zwei Aminfunktionen und mindestens eine aromatische C₆-C₁₄-Einheit, vorzugsweise C₆-C₁₀-Einheit, umfaßt, die gegebenenfalls mit einem oder mehreren linearen, zyklischen oder verzweigten, gesättigten oder ungesättigten C₁-C₁₀-Kohlenwasserstoffresten, vorzugsweise C₁-C₆-Kohlenwasserstoffresten, substituiert ist.

6. Verfahren nach dem vorhergehenden Anspruch, **dadurch gekennzeichnet, daß** man mindestens eine Verbindung (A) einsetzt, in der die aromatische Einheit gegebenenfalls mit einem oder mehreren Kohlenwasserstoffresten substituiert ist, die ausgewählt sind aus C₁-C₁₀-, vorzugsweise C₁-C₆-Alkyl-, -Aryl-, -Alkylaryl- und -Arylalkyl-Resten.

7. Verfahren nach einem der vorhergehenden Ansprüche, **dadurch gekennzeichnet, daß** man mindestens eine Verbindung (A) einsetzt, die dem Produkt der Formel
H₂N-R-NH₂ (1)
entspricht, wobei in der Formel (1) R eine aromatische Einheit darstellt, wie in einem der Ansprüche 4 oder 5 beschrieben.

8. Verfahren nach dem vorhergehenden Anspruch, **dadurch gekennzeichnet, daß** man mindestens eine Verbindung (A) der Formel (1) einsetzt, in der R eine aromatische C₆-C₁₄-Einheit, vorzugsweise C₆-C₁₀-Einheit, darstellt, die gegebenenfalls mit einem oder mehreren C₁-C₁₀-Alkylresten, vorzugsweise C₁-C₆-Alkylresten, substituiert ist.

9. Verfahren nach dem vorhergehenden Anspruch, **dadurch gekennzeichnet, daß** man mindestens eine Verbindung (A) einsetzt, die ausgewählt ist aus Toluoldiamin, Xylyloldiamin und Phenylendiamin, allein oder in Mischung, mit oder ohne ihre Isomeren.

10. Verfahren nach einem der vorhergehenden Ansprüche, **dadurch gekennzeichnet, daß** man die mindestens eine Verbindung (A) und/oder das Phosgen allein oder in Gegenwart eines Verdünnungsmittels einsetzt.

11. Verfahren nach dem vorhergehenden Anspruch, **dadurch gekennzeichnet, daß** man die Verbindung(en) (A) in einer Massenkonzentration von 3 bis 30 %, vorzugsweise 10 bis 20 %, in einem Verdünnungsmittel einsetzt.

12. Verfahren nach einem der vorhergehenden Ansprüche, **dadurch gekennzeichnet, daß** die Verweildauer der Reagenzien in dem Reaktor 1,5 bis 30 Sekunden beträgt.

## Claims

1. A process for preparing aromatic compounds substituted with at least two isocyanate groups, **characterised in that** at least one compound (A), containing at least two primary amine functions and at least one aromatic group, is brought into contact with phosgene, both reactants being introduced in the gaseous phase, in the presence of an excess of phosgene of between 0 and 100 % with respect to the number of moles of amine functions of compound (A), in a mixed reactor comprising a first perfectly stirred and cylindrical zone with a cross section close to that of its length, corresponding to 20% to 80% of the total volume of the reactor, and a second tubular zone in which the flow is close to piston flow, the length of which is longer than its cross section, corresponding to 80% to 20% of the total volume of the reactor.

2. A process for preparing aromatic compounds substituted with at least two isocyanate groups, **characterised in that** at least one compound (A), containing at least two primary amine functions and at least one aromatic group, is brought into contact with phosgene, both reactants being introduced in the gaseous phase, in the presence of an excess of phosgene of between 0 and 100 % with respect to the number of moles of amine functions of compound (A), in a mixed reactor comprising a first perfectly stirred zone, corresponding to 20% to 80% of the total volume of the reactor, and a second zone in which the flow is close to piston flow, corresponding to 80% to 20% of the total volume of the reactor; the residence time in one and the other of the zones being proportional to the volume distribution in each of the two zones.

3. A process according to claim 1 or claim 2, **characterised in that** the reaction is carried out in the presence of an excess of phosgene of between 5% and 60% with respect to the number of amine functions in compound (A).

4. A process according to any one of the preceding claims, **characterised in that** the reaction is carried out in a mixed reactor in which the first zone corresponds to 40% to 80% of the total volume of the reactor and the second zone corresponds to 60% to 20% of the total volume of the reactor.

5. A process according to any one of the preceding claims, **characterised in that** at least one compound (A) is used, containing at least two primary amine functions and at least one aromatic C₆-C₁₄ group, preferably C₆-C₁₀, which may or may not be substituted with one or more linear, cyclic or branched, saturated or unsaturated C₁-C₁₀ hydrocarbon radicals, preferably C₁-C₆.

6. A process according to the preceding claim, **characterised in that** at least one compound (A) is used in which the aromatic group can optionally be substituted with one or more hydrocarbon radicals selected from alkyl, aryl, alkyl-aryl and aryl-alkyl C₁-C₁₀ radicals, preferably C₁-C₆ radicals.

7. A process according to any one of the preceding claims, **characterised in that** at least one compound (A) is used which has formula:
H₂N-R-NH₂ (1)
in which formula (1), R represents an aromatic moiety as described in claim 4 or claim 5.

8. A process according to the preceding claim, **characterised in that** at least one compound (A) is used with formula (1) is used, where R represents an aromatic C₆-C₁₄ group, preferably C₆-C₁₀, which may or may not be substituted with one or more C₁-C₁₀ alkyl radicals, preferably C₁-C₆.

9. A process according to the preceding claim, **characterised in that** at least one compound (A) is used, selected from toluene diamine, xylylene diamine, or phenylene diamine, used alone or as a mixture, with or without their isomers.

10. A process according to any one of the preceding claims, **characterised in that** at least one compound (A) and/or phosgene is used, alone or in the presence of a diluent.

11. A process according to any one of the preceding claims, **characterised in that** the compound(s) (A) is/are used in concentration in the range 3% to 30% by weight, preferably in the range 10% to 20%, in the diluent.

12. A process according to any one of the preceding claims, **characterised in that** the residence time for the reactants in the reactor is between 1.5 and 30 seconds.
